(19) Europäisches Patentamt
European Patent Office
Office européen des brevets

(11) EP 0 943 296 A1

# (12) EUROPÄISCHE PATENTANMELDUNG

(43) Veröffentlichungstag:
**22.09.1999 Patentblatt 1999/38**

(51) Int. Cl.$^{6}$: **A61C 13/00**

(21) Anmeldenummer: **98811131.6**

(22) Anmeldetag: **12.11.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **17.03.1998 EP 98810230**

(71) Anmelder:
**Eidgenössische Technische Hochschule Zürich**
**8092 Zürich (CH)**

(72) Erfinder:
- **Filser, Frank, Dipl.-Ing.**
**8102 Oberengstringen (CH)**
- **Gauckler, Ludwig, Prof.-Dr.**
**8200 Schaffhausen (CH)**
- **Kocher, Peter**
**8304 Wallisellen (CH)**
- **Luethy, Heinz, Prof.-Ing.**
**2000 Neuchâtel (CH)**
- **Schaerer, Peter, Prof.-Dr.**
**8053 Zürich (CH)**

(74) Vertreter:
**Patentanwälte Breiter + Wiedmer AG**
**Seuzachstrasse 2**
**Postfach 366**
**8413 Neftenbach/Zürich (CH)**

## (54) Zahnkronen und/oder Zahnbrücken

(57) Die Erfindung bezieht sich auf ein Verfahren und einen Rohling zur Herstellung von auf wenigstens einen vorpräparierten Zahnstumpf (10) aufpassbarem künstlichem Zahnkronen (28) und/oder Zahnbrücken (38). Die dreidimensionale äussere und innere Oberfläche (20,22) eines Positivmodells (46) des Grundgerüstes (14) für die Zahnkronen (28) und/oder die Zahnbrücken (38) werden abgetastet und digitalisiert. Die ermittelten Daten werden um einen die Sinterschrumpfung exakt kompensierenden Faktor ( f ) in allen Raumrichtungen linear vergrössert, in die Steuerelektronik wenigstens einer Bearbeitungsmaschine für die Bearbeitung der Rohlinge (48) aus poröser Keramik übertragen und davon geeignete Werkzeugwege abgeleitet. Mittels Steuerbefehlen für Werkzeuge wird von der Digitalisierung zeitlich entkoppelt Material vom Rohling (48) abgetragen, bis eine vergrösserte Ausführungsform des Positivmodells (46,47) vorliegt. Dieses vergrösserte Grundgerüst wird zum Grundgerüst (14) mit direkten Endmassen dicht gesintert. Abschliessend wird das Grundgerüst (14) durch Verblenden mit Beschichtungsmaterial (24) aus Porzellan oder Kunststoff individualisiert. Auf dem Rohling aus poröser Keramik, seiner Verpackung, einer Anhängeetikette oder einem Beipackzettel ist ein maschinell oder mit menschlichen Sinnesorganen erfassbarer Informationscode für den Vergrösserungsfaktor (f) aufgebracht.

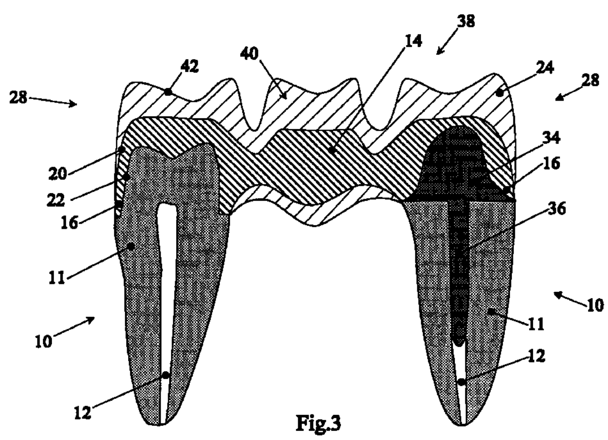

Fig.3

EP 0 943 296 A1

## Beschreibung

Die Erfindung bezieht sich auf ein Verfahren zur Herstellung von auf wenigstens einen vorpräparierten Zahnstumpf aufpassbarem künstlichem Zahnersatz, wobei unter Berücksichtigung der Schrumpfung aufgrund eines Modells ein vollkeramisches Grundgerüst aus biologisch verträglichem Material berechnet, durch Materialabtrag aus einem Rohling herausgearbeitet, dicht gesintert und zur Individualisierung ein Beschichtungsmaterial aufgebracht wird. Weiter betrifft die Erfindung einen Rohling aus poröser Keramik zur Durchführung des Verfahrens.

Es sind zahlreiche Methoden zur Herstellung von künstlichen Zahnkronen und/oder Zahnbrücken bekannt. Grundsätzlich wird nach der zahnärztlichen Präparation ein Abdruck des Zahnstumpfes/der Zahnstümpfe, der Zahnumgebung und des Kiefers angefertigt. Manuell kann über Gipsabformung ein Positivmodell der Situation im Mund hergestellt werden, auf dem mit handwerklichem Geschick ein Grundgerüst in Wachs oder Kunststoff modelliert wird. Bei Anwendung der konventionellen Technik wird über bekannte Verfahren, wie das Wachsausschmelzverfahren, 1:1 Kopierfräsen oder -schleifen, ein Modell des Grundgerüstes in Metall ausgeführt und mit Porzellan überbrannt. Neben dem hohen Ausschussrisiko beim Aufbrennen des Porzellans sind Abstriche in ästhetischer Hinsicht, vorallem am zervikalen Rand, hinzunehmen und röntgenbasierte Diagnoseverfahren zur Überwachung der überkronten Zähne nicht mehr anwendbar. In anderen Verfahren eingesetzte sogenannte dentalkeramische Porzellane sind aufgrund ihrer schlechten mechanischen Eigenschaften wohl für niedrig belastete Zahnkronen geeignet, für Zahnbrükken dagegen nicht einsetzbar.

In der EP,B1 0389461 wird ein Verfahren zur Herstellung eines künstlichen Zahnkronen-Onlays (onlay tooth crown) zum Einpassen in eine vorbereitete Zahnkavität beschrieben. Das Verfahren geht von einem Abdruck oder einer Negativform der Situation im Patientenmund aus. Die Zahnkronen-Onlays werden durch Kopierfräsen aus einem grünen oder vorgesinterten keramischen Rohling vergrössert herausgefräst und anschliessend dicht gesintert. Die Zahnkronen-Onlays gemäss der EP,B1 0389461 sind von Zahnkronen und Zahnbrücken grundsätzlich verschiedene Produkte, die zahnmedizinische Indikation ist eine andere. Zahnkronen-Onlays werden in Zahnhöhlungen eingepasst und sind bezüglich der geometrischen Form stets konvex geformt. Zahnkronen, auch mit Zahnbrücken, werden auf einen Zahnstumpf aufgepasst und haben die Form eines Käppchens. Dadurch ergeben sich dünn auslaufende Berandungen, welche technologisch schwierig zu handhaben sind. Ein wesentliches Merkmal des Kopierfräsens gemäss der EP,B1 0389461 ist die Zeitgleichheit von Abtasten und Übertragen der Abtastbewegung auf ein Bearbeitungswerkzeug. Im wesentlichen entspricht dies der Arbeitsweise eines Pantographen, welcher seit langem für lineare Zeichnungsvergrösserungen eingesetzt wird. Der Anwendungsbereich der EP,B1 0389461 ist deshalb auf ausschliesslich konvex geformte Zahnersatzprodukte beschränkt, wie beispielsweise Inlays, Onlay-Zahnkronen und Verblendungen.

Die EP,A2 580565 beschreibt künstliche Zahnrestaurationen mit pulvermetalurgischen Herstellungsverfahren eines keramisch dichten, hochfestem Grundgerüstes, welches mit Dentalporzellan beschichtet wird. Die Form der Zahnpräparation wird im Mund oder an einem Zahnmodell optisch oder mechanisch aufgenommen. Die Kavitäten, d.h. die innere Oberfläche, und ihre lokale Beziehung zueinander werden vergrössert aus einem Material, z.B. aus Kunststoff, mit einer rechnergesteuerten Fräsmaschine hergestellt. Die Kavitäten des Grundgerüstes werden in einem pulvermetallurgischen Prozess mit dieser Form hergestellt, also durch Pressen von Pulver über einen vorgefertigten, vergrösserten Zahnstumpf. Die äussere Oberfläche des Grundgerüstes wird ebenfalls durch Pressen gestaltet. Das Verfahren zur Herstellung des Grundgerüstes unterscheidet sich also grundsätzlich vom Herausarbeiten aus einem Rohling durch Materialabtrag.

Die Erfinder haben sich die Aufgabe gestellt, ein Verfahren der eingangs genannten Art zu schaffen, welches die Herstellung von vollkeramischen Zahnkronen und/oder Zahnbrücken mit einem Grundgerüst aus dicht gesinterter, hochfester Keramik zum Aufpassen und adhäsiven und/oder retentiven Befestigen auf natürlichen oder künstlichen Zahnstümpfen erlaubt. Das Verfahren soll die Herstellung von Zahnkronen und/oder Zahnbrücken mit okklusaler und kavitaler Oberfläche aus beim Sintern schrumpfenden Werkstoffen erlauben, welche auch bei filigraner Gestalt eine einwandfreie Passform haben, also keine Nacharbeit erforderlich machen. Weiter soll ein Rohling aus oxidkeramischem Material zur Verfügung stehen, der eine einfache, exakte Durchführung des Verfahrens erlaubt.

Die Aufgabe wird in bezug auf das Verfahren erfindungsgemäss dadurch gelöst, dass die dreidimensionale äussere und innere Oberfläche eines Positivmodells des Grundgerüstes für Zahnkronen und/oder Zahnbrücken abgetastet und digitalisiert werden, die Daten um einen die Sinterschrumpfung exakt kompensierenden Vergrösserungsfaktor f in allen Raumrichtungen linear vergrössert, in die Steuerelektronik wenigstens einer Bearbeitungsmaschine für die Bearbeitung der Rohlinge aus poröser Keramik übertragen und davon geeignete Werkzeugwege abgeleitet werden, von der Digitalisierung zeitlich entkoppelt, mittels Steuerbefehlen für Werkzeuge, von einem Rohling Material abgetragen wird, bis eine vergrösserte Ausführungsform eines Positivmodells vorliegt, welche zum Grundgerüst mit exakten Endmassen dicht gesintert wird, und dann durch Verblendung mit Beschichtungsmaterial aus Porzellan oder Kunststoff individualisiert

wird. Spezielle und weiterbildende Ausführungsformen des erfindungsgemässen Verfahrens sind Gegenstand von abhängigen Ansprüchen.

Ausgehend von einer zahnärztlichen Präparation der Zahnstümpfe wird ein Abdruck angefertigt, der ein Negativmodell der Situation im Mund des Patienten abformt, insbesondere die Oberfläche des Zahnstumpfes oder der Zahnstümpfe, die Approximalflächen der Nachbarzähne und der Gegenbiss. Weitergehend von dieser Abformung wird ein Positivmodell, meist aus Gips, hergestellt. Auf dem Positivmodell der Situation wird Abstandslack aufgetragen, welcher einen Spalt zwischen den Oberflächen des aufgrund des Modells hergestellten Grundgerüstes und des Zahnstumpfes berücksichtigt. Darauf kann auf dem bezeichneten Positivmodell der Situation im Patientenmund ein Modell für das Grundgerüst aus Wachs oder Kunststoff angefertigt werden. Dieses Vorgehen ist bekannt und wird in der zahntechnischen Praxis auch zur Herstellung von metallischen Grundgerüsten für Zahnkronen und/oder Zahnbrücken angewendet.

Das erfindungsgemässe Verfahren schliesst an diese an sich bekannte Vorstufe an und digitalisiert die äussere und innere Oberfläche des Grundgerüstmodells bzw. die Oberflächen am Positivmodell vollständig. Ein die Situation im Patientenmund unvollständig wiedergebendes Positivmodell wird vorzugsweise bezüglich der dreidimensionalen äusseren und inneren Oberfläche rechentechnisch ergänzt, was insbesondere im Bereich der Brückenglieder von Zahnbrücken von Bedeutung ist. Das Ergebnis der Digitalisierung und einer allfälligen rechentechnischen Ergänzung ist eine digitale Beschreibung der kompletten Oberfläche des Grundgerüstes. Die Digitalisierung kann mechanisch oder optisch erfolgen. Verfahren zur Digitalisierung im Mund eines Patienten auf einem präparierten Zahnstumpf oder an einem Modell sind beispielsweise aus der US,A 4182312 (mechanisch) und EP,B1 0054785 (optisch) bekannt. Der wesentliche Nachteil des bekannten mechanischen Digitalisierens liegt in der Fixierung des mechanischen Abtastgerätes am Patienten, die sichere Handhabung des Gerätes in der engen Mundhöhle ist problematisch. Bei optischen Digitalisiergeräten ist es notwendig, den Zahnstumpf aufgrund seiner transluszenten Eigenschaften mit Pulver zu beschichten, um Ungenauigkeiten durch teilweises und unkontrolliertes Eindringen von Licht in den zu vermessenden Zahnstumpf zu verhindern. Die Beschichtung mit einer Pulverschicht erhöht aber gleichzeitig auch die Ungenauigkeit durch das Auftragen einer meist ungleichmässigen Pulverhöhe auf den Zahnstumpf.

Im erfindungsgemässen Verfahren kann das Grundgerüstmodell mit Klemmstiften aufgespannt werden. Das aufgespannte Grundgerüstmodell wird zweckmässig in Schritten gedreht. Eine Drehung um 180° erlaubt eine vollständige Digitalisierung der okklusal und kavital zugänglichen Oberflächen des Grundgerüstmodells. Die optimalen Arbeitspositionen werden vorausbestimmt und durch Drehen der Wellen angesteuert.

Die Dimensionen der Oberfläche des Grundgerüstmodells werden zur Kompensation der Schrumpfung beim Sintern in allen Raumrichtungen linear vergrössert. Der Vergrösserungsfaktor f ergibt sich aus dem Raumgewicht $\rho_R$ des vorgefertigten Rohlings und dem erreichbaren Raumgewicht $\rho_S$ nach der Sinterung gemäss Gleichung 1

$$f = \sqrt[3]{\frac{\rho_S}{\rho_R}} \qquad (1)$$

Aus den Daten der vergrösserten Oberfläche werden die Steuerbefehle für die Bearbeitungsmaschine generiert, mit denen das vergrösserte Grundgerüst aus dem Rohling vollständig und vergrössert herausgearbeitet wird. Gegenüber der vergrösserten Oberfläche des Grundgerüstmodells wird kein Aufmass belassen, so dass bei der nachfolgenden Sinterschrumpfung direkt die exakten Endmasse erreicht werden, wodurch eine Nachbearbeitung im dicht gesinterten Zustand vermieden wird.

Zeitlich entkoppelt von der Digitalisierung kann nun ein Rohling aus poröser Keramik durch Materialabtrag zum vergrösserten Grundgerüst geformt werden. Dazu kann der Rohling z.B. zwischen zwei Wellen der Bearbeitungsmaschine aufgespannt werden. Nach einem abgeleiteten geeigneten Werkzeugweg wird der drehbar gelagerte Rohling bearbeitet. Die Bearbeitung kann mechanisch, beispielsweise mittels der Fertigungsverfahren Fräsen oder Schleifen mit einem oder mehreren Werkzeugen, und/oder optisch mit einem oder mehreren Strahlen erfolgen, beispielsweise durch Laserstrahlen. Die Bearbeitung kann in einem oder in verschiedenen Bearbeitungsschritten erfolgen, beispielsweise zuerst eine Rohbearbeitung, dann die Endbearbeitung der vom Werkzeug zugänglichen Oberflächen. Beim Wechsel von okklusaler zu kavitaler Bearbeitung kann ein Positionswechsel des teilbearbeiteten Rohlings notwendig sein. Das Drehen der Wellen mit dem gehalterten Rohling kann programmgesteuert schrittweise und/oder kontinuierlich erfolgen, mit insgesamt einer halben, einer ganzen oder mehreren Umdrehungen, auch mit Rückwärtsdrehungen.

Der Materialabtrag von einem Rohling erfolgt vorzugsweise mit Fräserwerkzeugen mit geometrisch bestimmten Schneiden, bei Drehzahlen im Bereich von vorzugsweise 10'000 bis 50'000 Touren/min., einer Zustellung von vorzugsweise > 0,5 mm, insbesondere von 1 - 15 mm, und einer Vorschubgeschwindigkeit von vorzugsweise > 3 cm/sec, insbesondere 3,5 - 10 cm/sec.

Die Herstellung des gegenüber dem Positivmodells vergrösserten Grundgerüstes aus dem Werkstoff des Rohlings wird das distale oder mesiale Abtrennen des Grundgerüstes von den Resten des Rohlings abgeschlossen. An den Trennstellen kann eine geringe manuelle Nacharbeit, ein sogenanntes Verputzen, notwendig sein.

Das fertig bearbeitete vergrösserte Grundgerüst wird dicht gesintert. Je nach verwendetem Material und Pulvermorphologie bewegen sich die Temperaturen in der Regel im Bereich von 1100 bis 1600°C. So kann eine Dichte von 90 bis 100% der theoretisch möglichen Dichte, vorzugsweise eine Dichte von 96 bis 100% der theoretisch möglichen Dichte, insbesondere mehr als 99% der theoretisch erreichbaren Dichte erreicht werden. Während des Sinterns schrumpft das Grundgerüst linear, ohne weitere Verformung oder Verzug. Dies erlaubt, den Sinterbrand ohne einen mitschwindenden Sinterstumpf durchzuführen. Die Schrumpfung S berechnet sich entsprechend der Gleichung (1) aus dem Raumgewicht des Rohlings $\rho_R$ vor der Sinterung und dem erreichbaren Raumgewicht $\rho_S$ nach der Sinterung:

$$S = \sqrt[3]{\frac{\rho_R}{\rho_S}}-1 \qquad (2)$$

Nach der Sinterung wird das geschrumpfte keramische Grundgerüst nach herkömmlichen Aufbrennverfahren bei Temperaturen von 700 bis 1100°C mit einer Beschichtung aus Porzellan, oder mit einer Beschichtung aus Kunststoff versehen. Es können eine oder mehrere Schichten aus Porzellan oder Kunststoff aufgebracht werden. Damit erhält die Zahnkrone oder Zahnbrücke ein individuelles Aussehen. Abschliessend wird die Zahnkrone oder Zahnbrücke mit Zement auf dem präparierten Zahnstumpf befestigt, wobei herkömmliche Zementierungswerkstoffe und Vorbereitungsprozeduren angewendet werden.

Die Vorteile des erfindungsgemässen Verfahrens können wie folgt zusammengefasst werden:

- Mit einem kostengünstigen, einfachen und sicheren Verfahren können qualitativ hochwertige und massgenaue, dicht gesinterte vollkeramische Zahnkronen und/oder Zahnbrücken hergestellt werden. Für ein sicheres und einfaches Herstellungsverfahren bilden homogene Rohlinge eine wesentliche Voraussetzung.
- Die individualisierten Zahnkronen und/oder Zahnbrücken zum Aufpassen auf präparierte Zahnstümpfe widerstehen den hohen Belastungen im Seitenzahnbereich und erfüllen die ästhetischen Ansprüche des Patienten im Frontzahnbereich gleichermassen. Insbesondere im Fall von Zahnbrükken besteht das Ziel in einer hohen Separation, d.h. mit einer grazilen Gestaltung zwischen den Brükkengliedern kann eine mit metallkeramischen Zahnbrücken wenigstens vergleichbare Gestaltung, die von Zahnärzten aus ästhetischen, hygienischen und phonetischen Gründen gefordert wird, erreicht werden.

Bezüglich des Rohlings aus poröser Keramik wird die Aufgabe erfindungsgemäss dadurch gelöst, dass auf dem Rohling selbst, seiner Verpackung, einer Anhängeetikette oder einem Beipackzettel ein maschinell oder mit menschlichen Sinnesorganen erfassbarer Identifikationscode mit Daten zur individuellen Eingabe des kompensierenden Vergrösserungsfaktors f aufgebracht ist.

Poröse Rohlinge aus Keramik für die Herstellung von Grundgerüsten für Zahnkronen und/oder Brükken können aus verschiedensten Materialkompositionen bestehen, insbesondere aus wenigstens einem Metalloxidpulver der Gruppe bestehend aus $Al_2O_3$, $TiO_2$, MgO, $Y_2O_3$ und Zirkonoxidmischkristall $Zr_{1-x}Me_xO_{2-(\frac{4-n}{2})x}$ bestehen, wobei Me ein Metall ist, das in Oxidform als 2-, 3- oder 4-wertiges Kation vorliegt (n = 2, 3, 4 und $0 \leq x \leq 1$) und die tetragonale und/oder kubische Phase des Zirkonoxids stabilisiert. Weitere Details der Materialkomposition von Rohlingen ergeben sich aus den abhängigen Verfahrensansprüchen 11 bis 13. Die Rohlinge können auch eine thermische Vorbehandlung erfahren, welche in den abhängigen Verfahrensansprüchen 6 bis 10 näher erläutert sind.

In jedem der Verfahrensschritte zur Herstellung eines Rohlings bestehen Toleranzen, z.B. Temperaturprofile und Temperaturschwankungen während der thermischen Vorbehandlung eines Rohlings.

Der Vergrösserungsfaktor f (Gleichung 1) für die Herausarbeitung der Grundgerüste aus Rohlingen ist aus den vorgenannten Gründen normalerweise keine Konstante. Auch wenn die Rohlingen aus ein- und demselben Material bestehen und auf denselben Fertigungseinrichtungen mit demselben Verfahren hergestellt werden, bleibt der Vergrösserungsfaktor f nicht konstant. Erfindungsgemäss können die Flexibilität im Material und die Fertigungstoleranzen erreicht werden, indem der individuelle Vergrösserungsfaktor f für jeden Rohling bestimmt und zusammen mit jedem Rohling ausgeliefert wird. Dies wird vorzugsweise dadurch realisiert, dass die Daten für den Vergrösserungsfaktor f optisch, elektromagnetisch oder mechanisch-taktil erfassbar auf dem Rohling selbst, seiner Verpackung, einer Anhängeetikette oder einem Beipackzettel aufgebracht ist.

Nach der einfachsten Variante sind die Daten für den Hersteller von Zahnkronen und/oder -brücken von Auge lesbar und können direkt oder über ein Hilfsprogramm zur Herstellung einer vergrösserten Ausführungsform eines Positivmodells für ein Grundgerüst ausgewertet werden.

Vorzugsweise wird jedoch ein an sich bekanntes Identifikationssystem eingesetzt, mit welchem die Daten für den Vergrösserungsfaktor f eingelesen und automatisch in Steuerbefehle für Werkzeuge umgewandelt werden.

## Ausführungsbeispiele

Ein Grundgerüst für eine Zahnbrücke zum Aufpassen auf eine zahnärztliche Präparation wird aus tetragonal stabilisiertem $ZrO_2$-Pulver hergestellt, welches 5,1 gew% $Y_2O_3$ und geringe Verunreinigungen von insgesamt weniger 0,05 gew% an $Al_2O_3$, $SiO_2$, $Fe_2O_3$ und $Na_2O$ enthält. Die Primärpartikelgrösse ist submikron, sie liegt bei etwa 0,3 µm. Die Presslinge werden isostatisch bei etwa 300 MPa gepresst und im Grünzustand wird die äusserste Materialschicht von weniger als 2 mm Dicke durch Drehen entfernt. Nach der Vorbearbeitung liegt der Durchmesser bei 22 mm und die Höhe bei 47 mm. Das Raumgewicht wird mit 3.185 g/cm$^3$ bestimmt. Der bearbeitete Pressling wird während etwa 120 min bei etwa 850°C vorgesintert. Nach dem Ausbrennen der Bindersubstanzen wird das Raumgewicht 3.089 g/cm$^3$ nach der Vorsinterung ermittelt.

Eine Abformung der Situation im Mund eines Patienten wird mit Siliconmasse angefertigt, insbesondere wird eine Negativform der präparierten Zahnstümpfe mit der Präparationsgrenze und den Approximalflächen der Nachbarzähne erzeugt. Durch die Abformung in einer Gipsmasse wird eine Positivform hergestellt. Zur Erzeugung eines Zementspaltes werden die beiden präparierten Zahnstümpfe auf der Oberfläche mit Distanzlack gleichmässig bestrichen und so die formgebende Oberfläche gebildet. Auf dieser Positivform der Situation im Mund des Patienten wird das Grundgerüst aus Wachs im Positiv modelliert.

Das Wachsmodell des Grundgerüstes wird zwischen zwei Wellen aufgespannt und dann vorerst schlangenlinienähnlich die okklusal zugänglichen Oberflächen, anschliessend - nach der Drehung des Wachsmodells um 180° - die kavital zugänglichen Oberflächen mechanisch digitalisiert. Das Ergebnis ist eine digitale Beschreibung der vollständigen Oberfläche des Grundgerüstes.

Die digitale Beschreibung wird linear um den gemäss Formel (1) berechneten Vergrösserungsfaktur f = 1.2512 gedehnt und davon Steuerungsbefehle für die Bearbeitungsmaschine unter Berücksichtigung der verwendeten Bearbeitungswerkzeuge für eine Grob- und eine Endbearbeitung des Grundgerüstes generiert und an die Bearbeitungsmaschine transferiert. Die Schaftfräswerkzeuge mit runder Stirnfläche haben einen Durchmesser von 3 mm für die Grobbearbeitung und einen Durchmesser von 1,5 mm für die Endbearbeitung. Der zwischen zwei Wellen gespannte, teilbearbeitete Rohling wird um 180° gedreht, so dass die Oberfläche des Grundgerüstes vollständig und vergrössert aus dem Rohling herausgearbeitet werden kann. Dann wird das Grundgerüst vom Restrohling getrennt, die Abtrennstellen am Grundgerüst durch Schleifen angeglichen und das Grundgerüst von Pulverresten sorgfältig gesäubert.

Das vergrösserte Grundgerüst aus $ZrO_2$ mit $Y_2O_3$ wird dann bei etwa 1500° C gesintert. Nach dem Sintern wird ein Raumgewicht von 6,050 g/cm$^3$ ermittelt, was praktisch 100% des maximal erreichbaren Raumgewichtes entspricht. Das bei der Sinterung um 20,07% geschrumpfte Grundgerüst kann ohne weitere Nachbearbeitung auf dem Positivmodell der Situation im Mund des Patienten aufgepasst werden.

Abschliessend wird das Grundgerüst durch Aufbrennen von Schichten von Porzellan bei Temperaturen zwischen 700 und 1100°C individualisiert und im Mund des Patienten mit Phosphatzement adhäsiv befestigt.

Anhand von in der Zeichnung dargestellten Ausführungsbeispielen, welche auch Gegenstand von abhängigen Patentansprüchen sind, wird die Erfindung näher erläutert. Es zeigen schematisch:

- Fig. 1 einen Längsschnitt durch einen natürlichen Zahnstumpf mit einer künstlichen Zahnkrone,
- Fig. 2 ein vergrössertes Detail von Bereich A gemäss Fig. 1,
- Fig. 3 einen Längsschnitt durch zwei Zahnstümpfe mit einer dreigliedrigen Zahnbrücke,
- Fig. 4 eine okklusale Ansicht des Grundgerüstes einer Zahnbrücke,
- Fig. 5 eine kavitale Ansicht des Grundgerüstes einer Zahnbrücke,
- Fig. 6 die Aufspannsituation eines Grundgerüstmodells für das Digitalisieren,
- Fig. 7 die Aufspannsituation für einen unbearbeiteten Rohling,
- Fig. 8 die Aufspannsituation vor dem Heraustrennen eines herausgearbeiteten Rohlings, und
- Fig. 9 die Aufspannsituation für das Digitalisieren eines Grundgerüstmodells einer Zahnkrone.

Ein in Fig. 1 dargestellter Zahnstumpf 10 hat eine Pulpa 12 für einen nicht dargestellten Nerv. Dieser Zahnstumpf ist natürlich und vital, nach anderen Ausführungsformen kann der Zahnstumpf 10 natürlich und avital oder künstlich auf einem Implantat aufgebaut sein. Der Zahnstumpf 10 weist keine Hinterschneidungen auf.

Auf den Zahnstumpf 10 ist ein Grundgerüst 14 aus dicht gesintertem Keramikmaterial aufzementiert. Dieses Grundgerüst 14 weist eine in Richtung eines Enamels 18 dünn auslaufende Berandung 16 auf, welche wesentlich schwieriger und filigraner herzustellen ist, als eine bekannte Onlay-Zahnkrone mit ausschliesslich konvexen Flächen. Die äussere Oberfläche 20 des Grundgerüstes 14 verläuft konvex und kann okklusal bearbeitet werden, was weitgehend dem Stand der Technik entspricht. Die konkave innere Oberfläche 22 des Grundgerüstes 14 wird kavital bearbeitet, was insbesondere mit Blick auf die dünn auslaufende Berandung 16 äusserst heikel ist. Mit der vorliegenden Erfindung kann dieses Problem auch beim Einsatz von vollkeramischem Material gelöst werden.

Zur Bildung einer künstlichen Zahnkrone wird Beschichtungsmaterial 24 auf das Grundgerüst 14 aufgetragen, bis die ursprünglich natürliche Zahnform wieder hergestellt ist. Mit dem Beschichtungsmaterial 24 wird das Grundgerüst 14 individualisiert, d.h. mit Porzellan oder Kunststoff verblendet.

Im vergrösserten Bereich A gemäss Fig. 2 ist deutlich erkennbar, dass beidseits des Grundgerüstes 14 aus dicht gesinterter Keramik weitere Schichten ausgebildet sind. In Richtung des Dentins 11 ist eine Zementschicht 26 zur adhäsiven Befestigung des Grundgerüstes 14 auf dem Zahnstumpf 10. Das Beschichtungsmaterial 24 ist lediglich als verhältnismässig dünne Schicht eingezeichnet, diese Schicht kann wesentlich dicker und mit formender Aussenfläche 42 ausgestaltet sein und so eine Zahnkrone 28 bilden.

Die Oberfläche 30 des natürlichen Zahnstumpfs 10 wird durch die zahnärztliche Präparation gebildet. Diese Oberfläche 30 verläuft bis zur Präparationsgrenze 32, auf welcher die dünn auslaufende Berandung 16 des Grundgerüstes 14 aufliegt.

Ein links gezeichnete Zahnstumpf 10 gemäss Fig. 3 entspricht weitgehend demjenigen von Fig. 1. Ein auf der rechten Seite von Fig. 3 gezeichneter avitaler Zahnstumpf 10 hat einen unteren Rest von Dentin 11, auf welchen ein künstlicher Zahnstumpf 34 aufgesetzt und in der leblosen Pulpa 12 über einen Stift 36 verankert ist. Auf die beiden Zahnstümpfe 10 ist eine dreigliedrige Zahnbrücke 38 mit einer Zementschicht 26 (Fig. 2) adhäsiv befestigt. Diese Zahnbrücke 38 umfasst zwei Zahnkronen 28 und ein Brückenglied 40, sie dient als Ersatz von verlorener Zahnsubstanz. Auf einem ebenfalls dreigliedrigen Grundgerüst 14 aus hochfester, dicht gesinterter Keramik ist durch Verblenden mit Beschichtungsmaterial 24 aus Porzellan oder Kunststoff individualisiert. Dieses Beschichtungsmaterial hat eine Aussenfläche 42, welche möglichst derjenigen der ursprünglichen natürlichen Zähne entspricht.

Nach einer nicht dargestellten Ausführungsform kann eine Zahnbrücke 38 mehr als zwei unterstützende Zahnstümpfe 10 und/oder mehrere Brückenglieder 40 haben. Wie bereits erwähnt, können die unterstützenden Zahnstümpfe 10 auch Implantate mit künstlichen Zahnstümpfen sein.

In der okklusalen Ansicht gemäss Fig. 4 ist ein dreigliedriges Grundgerüst 14 mit der äusseren konvexen Oberfläche 20 dargestellt, in Fig. 5 eine kavitale Ansicht dieses Grundgerüstes 14 einer Zahnbrücke mit der inneren konkaven Oberfläche 22.

Fig. 6 zeigt zwei coaxiale, synchron angetriebene Wellen 44 mit je einer stirnseitigen Verengung zu einem Klemmstift 45. Eingespannt ist ein dreigliedriges Grundgerüstmodell 46 einer Zahnbrücke 38 (Fig. 3) in okklusaler Ansicht. Die Wellen 44 mit den stirnseitigen Klemmstiften 45 sind in axialer Richtung verschiebbar und um einstellbare Winkel synchron drehbar. Nach dem Digitalisieren der okklusalen Seite des Grundgerüstmodells 46 werden die Wellen 44 um 180° gedreht und auch die kavitale Seite digitalisiert.

In Fig. 7 ist ein zwischen zwei Wellen 44 drehbar eingespannter Rohling 48 aus gepresstem Keramikpulver dargestellt. Direkt auf dem Rohling 48 ist ein maschinenlesbarer Informationscode C mit Daten für den Vergrösserungsfaktor f aufgebracht, im vorliegenden Fall ein elektromagnetisch oder optisch lesbarer Strichcode. Der Informationscode C dient beispielsweise der Identifikation.

Für die Herstellung des Rohlings 48 werden Pulver oder Kolloide über bekannte Methoden der keramischen Formgebung zu grünen Rohlingen verarbeitet. Bekannte Verfahren zur Herstellung keramischer Grünkörper sind beispielsweise in der WO 94/02429 und 94/24064 beschrieben. Aus fertigungstechnischen Gründen werden für die Rohlinge geometrisch einfache Gestaltformen, wie Zylinder oder Quader, bevorzugt.

Vor der Vorbearbeitung kann der Rohling 48 einer Wärmebehandlung unterzogen werden. Diese erfolgt bevorzugt bei Temperaturen im Bereich von 50 bis 200°C, insbesondere von 90 bis 150°C, für eine Zeitdauer von bevorzugt 2 bis 20 Stunden, insbesondere von 2 bis 6 Stunden. Unmittelbar anschliessend kann der Rohling 48 materialabtragend zum vergrösserten Grundgerüst 14 weiterverarbeitet werden.

Die äussere Schicht 50 wird insbesondere abgetragen, wenn der Rohling 48 über Press-, Giess- oder Spritzverfahren hergestellt ist, um vorhandene Dichtegradienten in der äussersten Materialhülle abzutragen. Weitere übliche Herstellungsverfahren für Rohlinge 48 sind kaltisostatisches Pressen, uniaxiales Pressen, Schlickerguss, Druckguss, Spritzguss, Extrudieren, Rollen und DCC (direct coagulation casting).

Vor dem Weiterverarbeiten zum vergrösserten Grundgerüst 14 kann der Rohling 48 eine Vorsinterung erfahren, welche vorzugsweise während 0,5 bis 6 Stunden bei einer Temperatur von mindestens etwa 450°C, insbesondere in einem Temperaturbereich von 600 bis 1200° C, durchgeführt wird.

Die Rohlinge werden in der Praxis meist aus einem Metalloxidpulver der Gruppe bestehend aus $Al_2O_3$, $TiO_2$, MgO, $Y_2O_3$ und Zirkonoxidmischkristall

$$Zr_{1-x}Me_xO_{2-\left(\frac{4n}{2}\right)\cdot x}$$

hergestellt, wobei Me ein Metall ist, das in Oxidform als zwei-, drei- oder vierwertiges Kation vorliegt und die tetragonale und/oder die kubische Phase des Zirkonoxids stabilisiert. Bei der Formel für Zirkonmischoxidkristall ist n = 2, 3 oder 4, weiter gilt $0 \leq x \leq 1$.

Nach einer speziellen Ausführungsform wird das Metalloxidpulver mit einem organischen Binder vermischt, vorzugsweise aus wenigstens einer der Klassen Polyvinylalkohole (PVA), Polyacrylsäuren (PPA), Zellulosen, Polyethylenglukole und/oder Thermoplaste. Zweckmässig liegt der Binderanteil im Bereich von 0,1 bis 45 vol%, vorzugsweise im Bereich von 0,1 bis 5vol%.

Fig. 8 zeigt in okklusaler Ansicht die Aufspannsituation nach der Bearbeitung,
jedoch vor dem Heraustrennen des vergrösserten Grundgerüstes 14 von den verbliebenen Resten 52 des Rohlings 48.

In Fig. 9 wird in kavitaler Ansicht die Aufspannsituation für das Digitalisieren eines Grundgerüstmodells 47 für eine Zahnkrone gezeigt.

## Patentansprüche

1. Verfahren zur Herstellung von auf wenigstens einen vorpräparierten Zahnstumpf (10) aufpassbarem künstlichem Zahnersatz (28,38), wobei unter Berücksichtigung der Schrumpfung aufgrund eines Modells ein vollkeramisches Grundgerüst aus biologisch verträglichem Material berechnet, durch Materialabtrag aus einem Rohling herausgearbeitet, dicht gesintert und zur Individualisierung ein Beschichtungsmaterial (24) aufgebracht wird,
dadurch gekennzeichnet, dass

   die dreidimensionale äussere und innere Oberfläche (20,22) eines Positivmodells (46, 47) des Grundgerüstes (14) für Zahnkronen (28) und/oder Zahnbrücken (38) abgetastet und digitalisiert werden, die Daten um einen die Sinterschrumpfung exakt kompensierenden Vergrösserungsfaktor ( f ) in allen Raumrichtungen linear vergrössert, in die Steuerelektronik wenigstens einer Bearbeitungsmaschine für die Bearbeitung der Rohlinge (48) aus poröser Keramik übertragen und davon geeignete Werkzeugwege abgeleitet werden, von der Digitalisierung zeitlich entkoppelt, mittels Steuerbefehlen für Werkzeuge, von einem Rohling (48) Material abgetragen wird, bis eine vergrösserte Ausführungsform eines Positivmodells (46,47) vorliegt, welche zum Grundgerüst (14) mit direkten Endmassen dicht gesintert wird, und dann durch Verblenden mit Beschichtungsmaterial (24) aus Porzellan oder Kunststoff individualisiert wird.

2. Verfahren nach Anspruch 1, dadurch gekennzeichnet, dass ein die Situation im Patientenmund unvollständig wiedergebendes Positivmodell (46,47) bezüglich der dreidimensionalen äusseren und/oder inneren Oberfläche (20,22) rechentechnisch ergänzt wird, insbesondere im Bereich der Brückenglieder (40) von Zahnbrücken (38).

3. Verfahren nach Anspruch 1 oder 2, dadurch gekennzeichnet, dass der Vergrösserungsfaktor ( f ) des Positivmodells (46,47) eines Grundgerüstes (46) aufgrund der Materialzusammensetzung und der Pulvereigenschaften festgelegt wird, vorzugsweise nach der Formel

$$f = \sqrt[3]{\frac{\rho_S}{\rho_R}}$$

wobei $\rho_R$ das Raumgewicht des vorgefertigten Rohlings und $\rho_S$ das nach der Sinterung erreichbare Raumgewicht bedeuten.

4. Verfahren nach einem der Ansprüche 1 bis 3, dadurch gekennzeichnet, dass die Zahnkronen (28) und/oder Zahnbrücken (38) mit dünn auslaufender Berandung (16) ausgebildet werden.

5. Verfahren nach einem der Ansprüche 1 bis 4, dadurch gekennzeichnet, dass das bearbeitete, vergrösserte Grundgerüst (14) gesintert wird zu einer Dichte $\rho_S$ von 90 bis 100% der theoretisch möglichen Dichte, vorzugsweise zu einer Dichte $\rho_S$ von 96 bis 100% der theoretisch möglichen Dichte, insbesondere zu einer Dichte $\rho_S$ von über 99% der theoretisch möglichen Dichte.

6. Verfahren nach einem der Ansprüche 1 bis 5, dadurch gekennzeichnet, dass ein grüner oder vorgesinterter Rohling (48) aus gepresstem, feinem Keramikpulver eingesetzt wird, wobei eine Vorsinterung vorzugsweise erst nach dem Abtragen der äussersten Materialschicht (50) erfolgt.

7. Verfahren nach einem der Ansprüche 1 bis 6, dadurch gekennzeichnet, dass der Rohling (48) mechanisch und/oder optisch bearbeitet wird, wobei vorzugsweise zuerst eine Rohbearbeitung, dann eine Endbearbeitung erfolgt.

8. Verfahren nach einem der Ansprüche 1 bis 7, dadurch gekennzeichnet, dass der Rohling (48) vor der Vorbearbeitung einer Wärmebehandlung bei Temperaturen im Bereich von 50 bis 200°C, vorzugsweise von 90 bis 150°C, unterworfen wird, für eine Zeitdauer von 2 bis 20 Stunden, vorzugsweise von 2 bis 6 Stunden.

9. Verfahren nach Anspruch 8, dadurch gekennzeichnet, dass der Rohling (48) nach der Wärmebehandlung materialabtragend zum vergrösserten Grundgerüst (14) weiterverarbeitet wird.

10. Verfahren nach einem der Ansprüche 1 bis 9, dadurch gekennzeichnet, dass der Rohling (48) vor der Weiterverarbeitung zum vergrösserten Grundgerüst (14) eine Vorsinterung für 0.5 bis 6 Stunden bei einer Temperatur von mindestens etwa 450°C erfährt, die vorzugsweise im Bereich von 600 bis 1200°C liegt.

**11.** Verfahren nach einen der Ansprüche 1 bis 10, dadurch gekennzeichnet, dass ein Rohlinge (48) aus wenigstens einem der Metalloxidpulver der Gruppe bestehend aus $Al_3O_3$, $TiO_2$, MgO, $Y_2O_3$ und Zirkonoxidmischkristall

$$Zr_{1-x}Me_xO_{2-(\frac{4-n}{2})\,x}$$

eingesetzt wird, wobei Me ein Metall ist, das in Oxidform als zwei-, drei- oder vierwertiges Kation vorliegt (n= 2, 3, 4 und $0 \leq x \leq 1$) und die tetragonale und/oder die kubische Phase des Zirkonoxids stabilisiert.

**12.** Verfahren nach einem der Ansprüche 1 bis 11, dadurch gekennzeichnet, dass Metalloxidpulver mit einem organischen Binder eingesetzt werden, vorzugsweise aus wenigstens einer der Klassen Polyvinylalkohole (PVA), Polyacrylsäuren (PAA), Zellulosen, Polyethylenglukole und/oder Thermoplaste.

**13.** Verfahren nach Anspruch 12, dadurch gekennzeichnet, dass der Binderanteil im Bereich 0.1 bis 45 vol% liegt, vorzugsweise im Bereich 0.1 bis 5 vol%.

**14.** Rohling (48) aus poröser Keramik zur Durchführung des Verfahrens nach einem der Absprüche 1 bis 13,
dadurch gekennzeichnet, dass

auf dem Rohling (48) selbst, seiner Verpackung, einer Anhängeetikette oder einem Beipackzettel ein maschinell oder mit menschlichen Sinnesorganen erfassbarer Informationscode (C) mit Daten zur individuellen Eingabe des kompensierenden Vergrösserungsfaktors (f) aufgebracht ist.

**15.** Rohling (48) nach Anspruch 14, dadurch gekennzeichnet, dass der Identifikationscode (C) optisch, elektromagnetisch oder mechanisch-taktil erfassbar aufgebracht ist.

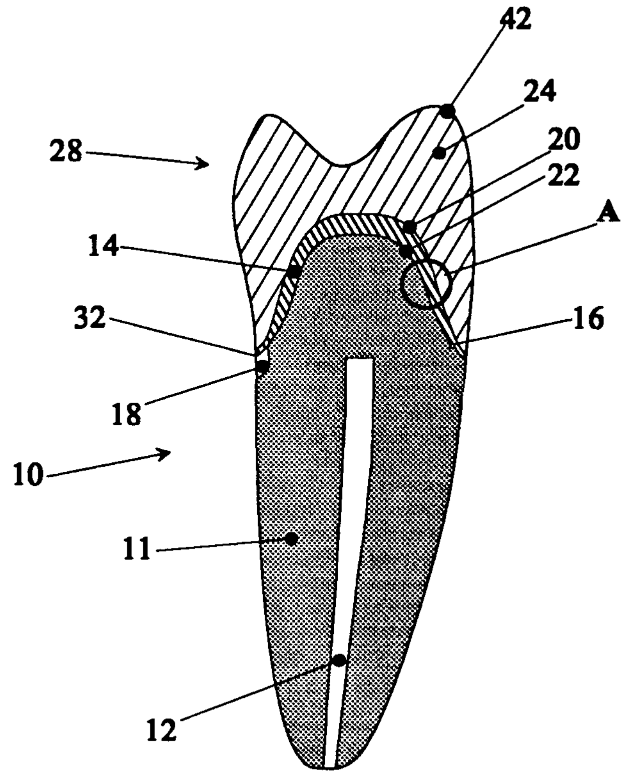


Fig.1

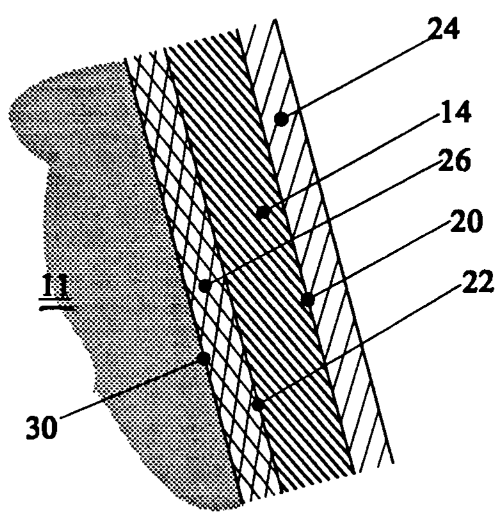


Fig.2

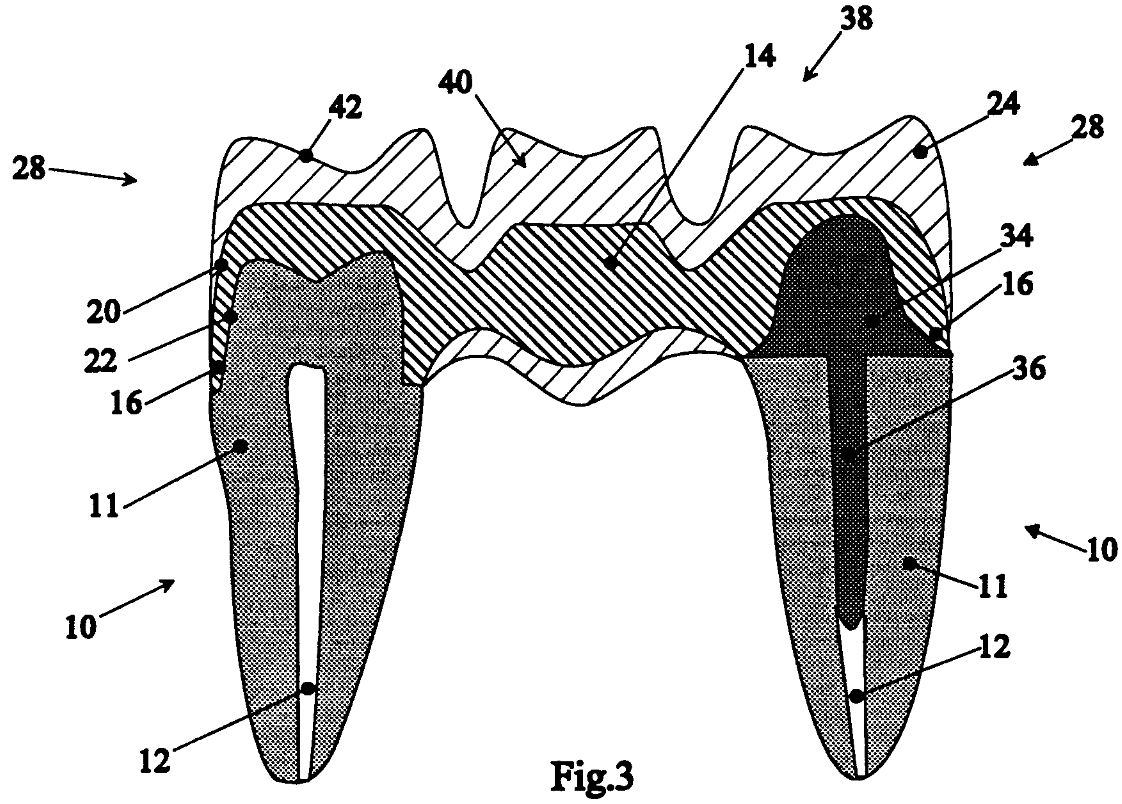


Fig.3

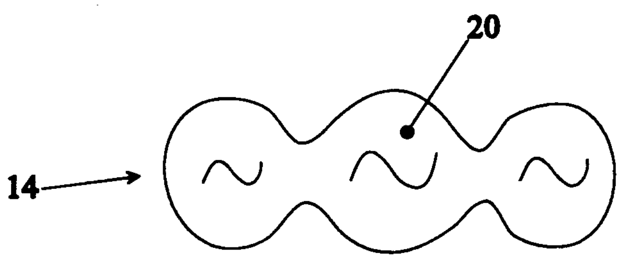


Fig.4

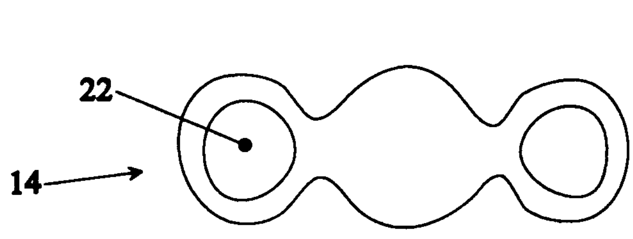


Fig.5

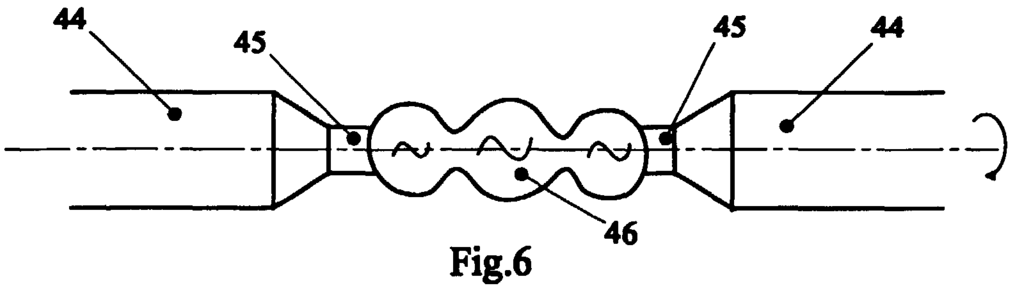

Fig.6

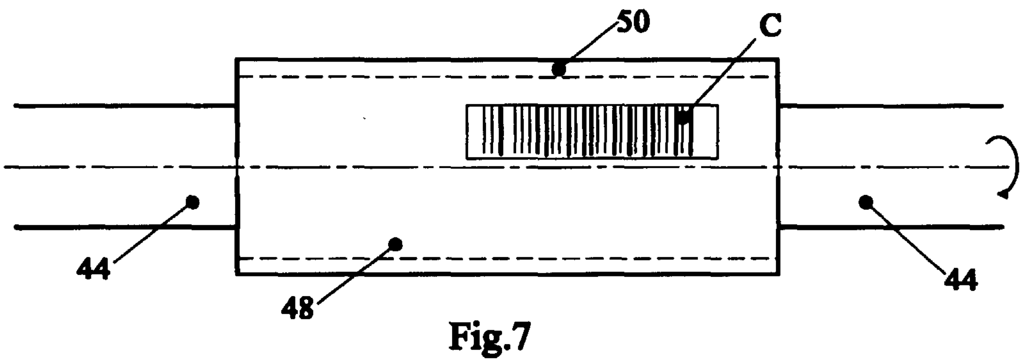

Fig.7

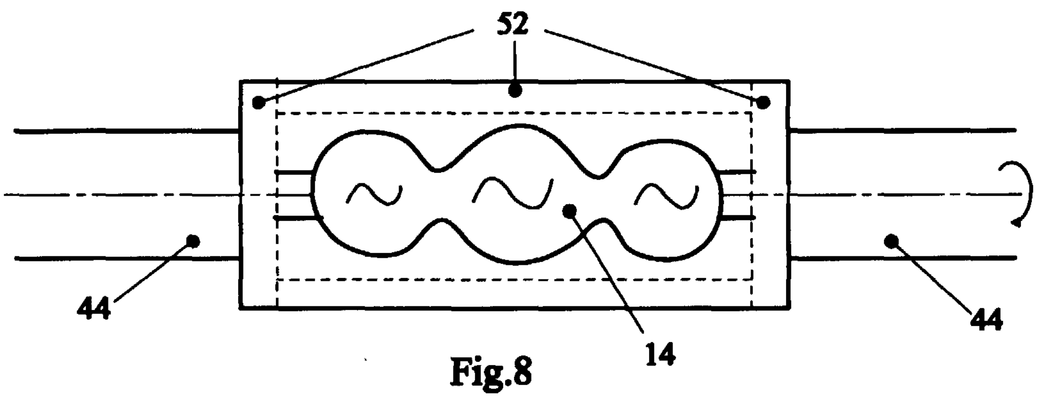

Fig.8

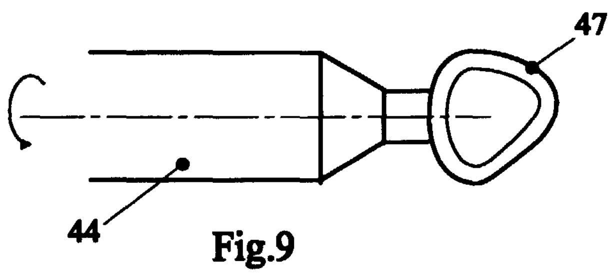

Fig.9

Europäisches Patentamt

# EUROPÄISCHER RECHERCHENBERICHT

Nummer der Anmeldung
EP 98 81 1131

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,X | EP 0 580 565 A (SANDVIK)<br>26. Januar 1994 (1994-01-26)<br>* das ganze Dokument *<br>--- | 1-7,<br>10-12 | A61C13/00 |
| X | EP 0 160 797 A (MÖRMANN)<br>13. November 1985 (1985-11-13)<br>* Seite 5, Zeile 10 - Zeile 13 *<br>----- | 14 | |

RECHERCHIERTE SACHGEBIETE (Int.Cl.6)
A61C

Der vorliegende Recherchenbericht wurde für alle Patentansprüche erstellt

| Recherchenort | Abschlußdatum der Recherche | Prüfer |
|---|---|---|
| DEN HAAG | 13. Juli 1999 | Vanrunxt, J |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 98 81 1131

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-07-1999

| Im Recherchenbericht angeführtes Patentdokument | Datum der Veröffentlichung | Mitglied(er) der Patentfamilie | Datum der Veröffentlichung |
|---|---|---|---|
| EP 580565 A | 26-01-1994 | SE 470346 B | 31-01-1994 |
| | | AT 170069 T | 15-09-1998 |
| | | DE 69320563 D | 01-10-1998 |
| | | DE 69320563 T | 14-01-1999 |
| | | ES 2119881 T | 16-10-1998 |
| | | FI 932910 A | 24-12-1993 |
| | | NO 932298 A | 27-12-1993 |
| | | SE 9201927 A | 24-12-1993 |
| | | US 5342201 A | 30-08-1994 |
| EP 160797 A | 13-11-1985 | CH 665551 A | 31-05-1988 |
| | | AT 39613 T | 15-01-1989 |
| | | JP 2013948 C | 02-02-1996 |
| | | JP 7028878 B | 05-04-1995 |
| | | JP 60215354 A | 28-10-1985 |
| | | US 4615678 A | 07-10-1986 |

EPO FORM P0461

Für nähere Einzelheiten zu diesem Anhang : siehe Amtsblatt des Europäischen Patentamts, Nr.12/82